# EUROPEAN PATENT APPLICATION

(11) **EP 0 730 874 A2**
(43) Date of publication of application: **11.09.1996**
(21) Application number: 96200173.1
(22) Date of filing: 24.01.1996
(51) Int. Cl.: A61L 15/58

(54) **Closure tape for improved wound or incision healing and its use**

(30) Priority: 24.01.1995 US 378133
(71) Applicant: Bristol-Myers Squibb Company, New York, N.Y. 10154 (US)
(72) Inventor: Constantine, Barry, Island Heights, New Jersey 08732 (US)
(74) Representative: Mays, Julie

(57) **Abstract**

The invention provides the use of a tape comprising : a polymeric elastic film that is substantially impermeant to microorganisms, and a hydrocolloid adhesive coating on one face of the film and having fluid absorbing capacity, for the manufacture of a closure tape for use in closing a breach of the skin of the type that has typically been closed with sutures. The invention also provides a closure tape adapted for the use according to the invention..

## Description

This invention relates to the discovery that elastic, film-backed wound dressings, when coated with a hydrocolloid adhesive, may be used to close incisions or other skin breaches usually requiring sutures, and that these dressings may be applied to cover the entire surface of a treated skin breach. Such incision coverings avoid the creation of suture tracks that can lead to infection, provide a barrier blocking access by infectious agents to an incision site, have sufficient elasticity to avoid the torsion blistering that often accompanies the use of acrylic adhesive tape closure strips, such as Steri-Strips® and similar inelastic wound dressings, and avoid the foreign body reaction that often accompanies the use of tissue glue.

The medical community has for a number of years had available to it flexible, elastic wound coverings coated with hydrocolloid adhesives. See, for instance, U.S. Patents 4,551,490, 4,538,603, and 4,909,243. Despite this availability, such coverings have not been used as incision closures. Instead the medical community has used sutures, surgical staples, tissue glues and small inelastic adhesive strips. The failure of the medical community to turn to flexible, elastic coverings for use as incision closures probably reflects the prevailing belief that incisions should be open to the air. One concern behind this belief is that by more fully covering the incision, as would result with flexible, elastic coverings, the exudate from the incision would remain moist and provide a fertile breeding ground for microorganisms.

Acrylic tape closure devices function essentially as substitutes for sutures. They are intended to clasp together discrete segments of a breach of the skin, leaving substantial area between each tape closure devices where exudate from the wound is not flow restricted by a closure device. Such devices do not absorb substantial amount of fluid such as exudate. The exudate emerging immediately below a non-absorbent adhesive device may travel, without substantial restriction, along the incision or breach to an area not covered with a closure device. Just as with sutures, an absorbent dressing is usually packed above the closure devices to absorb exudate. Being inelastic, acrylic closure devices often create torsion blisters when the wounded tissue swells and contracts due to normal motion and flexing of the skin, inflammation and the healing process.

It is believed that there has been an attempt to use acrylic tape closure devices, such as Steri-Strip® tapes (3M Corp., Saint Paul, Minnesota) in conjunction with a covering of a acrylic film dressing such as Tega-Derm® film (3M Corp.). The closure tapes were first used to close a wound. Then, the acrylic film was placed over the entire wound. However, fluid buildup under the dressing lead to pressure buildup, tissue maceration, increased infection potential and disruption of the acrylic adhesive, which processes aggravated the scarring process.

Sutures create "suture tracks" that create a pathway for microbes to breach the protective barrier of the skin and enter the subcutaneous tissue, thus increasing the potential for infection. With suturing, the inflammation associated with responding to increased microbial attacks, the foreign body reactions against the non-absorbable sutures, and the damage due to tissue strangulation created by the sutures are believed to aggravate the healing process. Surgical staples, while convenient and useful to reduce the time that a patient spends in surgery, have these same deficiencies.

When incisions or other breaches are treated with tissue glue, it is virtually impossible to prevent the flow of some of the glue into the incision or breach. This creates the potential for a foreign body reaction. Also, epithelialization can be blocked by the insoluble polymers of the glue, leading to epidermal detour wherein epidermal layers form on the sides of incision or breach.

It has been found, first, that flexible, elastic coverings, when coated with an appropriate hydrocolloid adhesive, can close skin breaches of the types that are usually treated with sutures without interference by wound exudate. More importantly, such incision closures have been found to allow an incision or skin breach to heal with an unexpected reduction in inflammation, swelling and scarring.

The invention relates to the use of a tape comprising :
(i) a polymeric elastic film that is substantially impermeant to microorganisms, and
(ii) a hydrocolloid adhesive coating on one face of the film and having fluid absorbing capacity,
for the manufacture of a closure tape for use in closing a breach of the skin of the type that has typically been closed with sutures. In this way, the invention provides for tissue apposition.

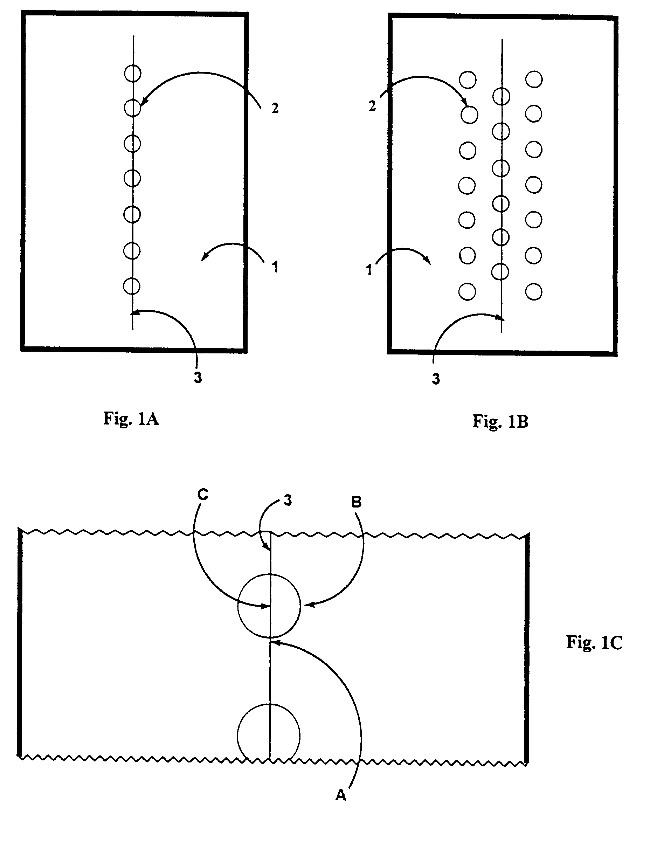

In an embodiment of the invention, the elastic film set forth above may possess a series of holes positioned to substantially align with the skin breach. The segments of the skin breach under the holes lacks the direct adhesive and bridging support provided to adjacent segments of the breach by the adhesive-coated film. However, the size of the holes is selected to be small enough so that the breach under the holes is substantially held together by the stabilizing action of the adjacent adhesive-coated film and the integument surrounding the breach. By reference to Fig. 1C, which is an enlargement of a portion of the dressing of Fig. 1A, one can see that the adhesive at positions A and B will add support for maintaining the apposition of the tissue at the segment of the breach at position C. This embodiment is useful for wounds that are anticipated to produce large amounts of exudate, especially early in the healing process (for instance, a wound caused by a procedure to remove a cyst). The holes provide a pathway for some of the exudate to exit from under the tape. An absorbent dressing may be placed immediately over the holes in the tape to absorb exudate. This absorbent dressing may be facilely replaced without disturbing the closed breach.

### Brief Description of the Drawings

Figures 1A, 1B and 1C illustrate the tape used in the invention when it has been applied to a skin breach.

Figures 2A and 2B illustrate an embodiment of the tape used in the invention when it has been applied to a skin breach.

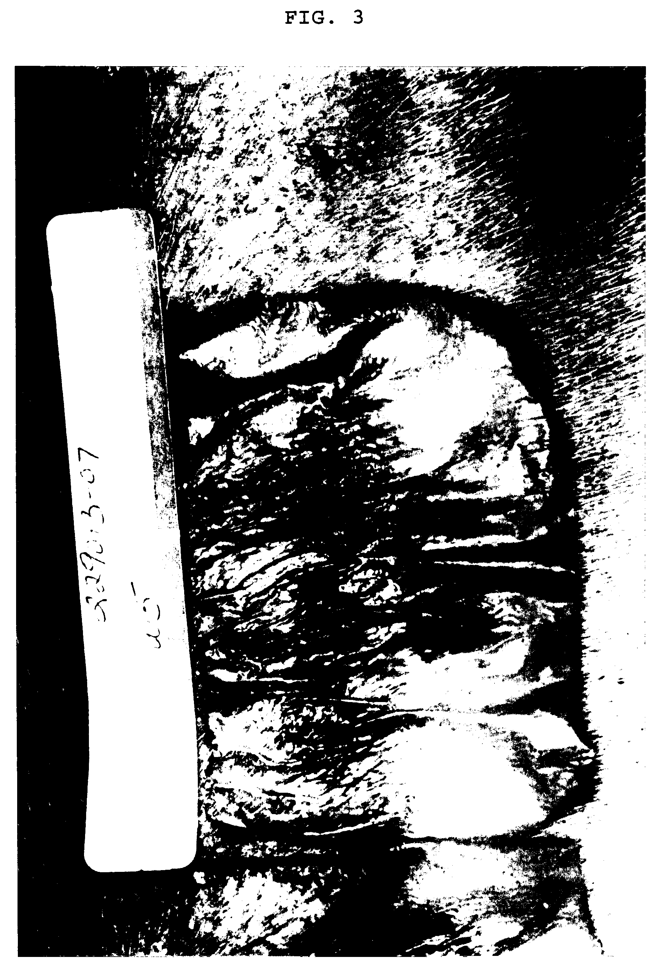

Figure 3 displays an incision that is closed and remains covered with a hydrocolloid closure tape.

Figure 4 displays a healed incision that was closed with a hydrocolloid closure tape. The closure device is also shown after it was partially pealed away from the skin surrounding the incision.

Figure 5 displays a healed incision that had been closed with a hydrocolloid closure tape.

Figure 6 displays an incision that was treated by closing with sutures and covering with a hydrocolloid dressing.

Figure 7 displays an incision that was treated with a tissue glue.

The polymeric elastic film can be made of a number of polymers, including polyurethane, polyacrylate, polyethylene and mixtures thereof. Preferably, the film comprises polyurethane. The modulus of elasticity of the film generally should be such that when the film is stretched 10% in the machine direction, it will exhibit a force of between about 1000 and about 5000 psi; preferably between about 2000 and about 4000; more preferably between about 2500 and about 3500. In this way, the elastic modulus of the film is not dissimilar to that of skin. The thickness of the film will generally be between about 0.0005 and about 0.003 inches (i.e., between about 1/2 and 3 mils), preferably between about 0.001 and about 0.002 inches, more preferably about 0.001 inches.

In a preferred embodiment, the surface of the film not coated with adhesive will be surface treated with a siliconizing agent such as polysiloxane (available from General Electric, Schenectady, NY). Generally, about 0.2 g/m² to about 0.6 g/m² of silicone will be applied, preferably, about 0.3 g/m². This surface treatment allows the closure tape to be rolled up on itself without the adhesive layer adhering too strongly to the non-adhesive surface upon it has been rolled. In the absence of such surface treatment, generally the closure tape will be stored loosely adhered to another siliconized layer material such a paper, so that it may be conveniently handled.

The hydrocolloid adhesives used in the invention incorporate (1) hydrophilic components and (2) hydrophobic components. The hydrophilic components typically include water soluble hydrocolloids such as sodium carboxymethylcellulose ("CMC"), pectin, gelatin, guar gum, locust bean gum, gum karaya, and mixtures thereof. Optional further hydrophilic components include water swellable adhesive strengthening agents, such as the following finely divided substantially water insoluble cross-linked materials: sodium CMC; starch-acrylonitrile graft copolymer; or dextran, as discussed in U.S. Patent No. 4,551,490, (the "'490 patent"), the entire disclosure of which is incorporated herein by reference.

The hydrophobic components (2) typically include one or more elastomers such as a polyisobutylene. Other typical hydrophobic components are elastomers such as a butyl rubber, styrene radial or block-type copolymers. Finally the hydrophobic components typically include an oil such as mineral oil. Examples of these components are described in the '490 patent.

Examples of hydrocolloid adhesives useful in the invention are described in the '490 patent, as are methods for mixing the component ingredients. The '490 patent further identifies component ratios believed to produce useful adhesives. Also identified in the '490 patent are optional components such as antimicrobial agents that may be added to the adhesive composition.

For the purpose now contemplated, the adhesive composition should be capable of absorbing substantial fluid while retaining adhesiveness. For this purpose, the adhesive preferably includes from about 5% to about 30% by weight of one or more low molecular weight polyisobutylenes or a blend of one or more low molecular weight polyisobutylenes and butyl rubber (more preferably about 5% to about 15%), from about 3% to about 20% by weight of one or more styrene-isoprene-styrene or styrene-butadiene-styrene block type copolymers (more preferably about 10% to about 20%), from about 8% to about 40% by weight of mineral oil (more preferably about 20% to about 40%), from about 15% to about 65% by weight of one or more water soluble hydrocolloid gums (more preferably about 30% to about 65%), up to about 15% by weight of one or more water swellable cohesive strengthening agents, provided that the water soluble hydrocolloid gums and cohesive strengthening agents combined are present at from about 15% to about 65% by weight of the composition (more preferably from about 30% to about 65% by weight of the composition), and from about 7.5% to about 15% by weight of a tackifier (more preferably from about 7.5% to about 10%). Optionally, the composition includes an antioxidant such as zinc dibutyldithiocarbamate or tetrakis (methylene--3,5-di-tertbutyl-4-hydroxyhydrocinnaminate)methane. Preferably, the antioxidant comprises up to about 0.5% of the composition.

Important considerations in selecting these component ratios are (1) the durability of the bond between the adhesive and the film and (2) the cohesiveness of the adhesive composition. If these are deficient, the hydrocolloid adhesive will have too strong a tendency to separate from the film after the film has been applied to a skin breach. This separation is unsightly, and creates an appearance not dissimilar from that of an infected pus. Accordingly, it is preferable to avoid such separation from the film. It is believed that sufficient hydrophobic components, particularly the polymers, should be incorporated into the adhesive to encapsulate the hydrophilic components, and thereby to avoid having these components separate from the film when they swell during use.

Preferably, the hydrocolloid adhesive is capable of swelling to incorporate at least about 0.03 g of water per mil of thickness and cm² of area while retaining adhesive tack, more preferably, it retains at least about 0.05 g of biological fluid per mil of thickness and cm² of area while retaining adhesive tack. Preferably, the hydrocolloid adhesive can retain at least about 0.3 g of water, more preferably at least about 0.5 g. It is not critical that a portion of the adhesive adjacent to the skin breach has lost its tack, since neighboring portions of the adhesive will generally retain adhesive tack and maintain tissue apposition.

The hydrocolloid adhesive coating will preferably have a thickness of from about 1.0 mils to about 100 mils, more preferably from about 5 mils to about 50 mils, still more preferably from about 5 mils to about 40 mils. The hydrocolloid adhesive can be tested for adhesiveness according to ASTM protocol D-3330, which measures the initial force needed for a 180° peel from stainless steel. Prior to absorbing fluid, the adhesiveness will generally be at least about 10 newtons, preferably at least about 12 newtons, more preferably at least about 14 newtons.

In a preferred embodiment, the film of the closure tape of the invention has a series of holes situated to align with the breach that is to be closed with the closure tape, as illustrated in Figure 1. These closure tapes should be useful for skin breaches that are anticipated to produce substantially exudate, especially in the first day or two after the tape is applied. Such high exudate producing breaches include, for example, breaches formed as a result of cyst removals. (Large amounts of exudate will generally flow into the subdermal void formed by the removal of a cyst and subsequently flow outward through the channels formed by surgical incisions.)

Figures 1A, 1B and 1C display closure tapes (1) with holes (2). The tapes are positioned on wounds to substantially align the holes with the lines (3) created by the closed skin breach. The use of a patterned arrangement of holes, as in Figure 1B, allows a covered skin breach to be more facilely aligned with the holes, especially when the breach does not define a straight line.

The holes should be of a size that allows for substantial exudate to flow out from beneath the closure tape but does not substantially disrupt the tissue clamping action of the closure tape. As the holes become too large, the sides of the breach under the hole will lose the structural support that holds them adjacent to one another. It is believed that this loss of support would lead to elliptical scar formation, epidermal detour and scarring. To avoid this, the size and placement of the holes is selected so that the tissue stabilization action of the tape is not substantially disrupted. Generally, the holes are sized and situated so that at least about 50% of the breach is covered by the hydrocolloid coated tape, preferably at least about 70%, more preferably at least about 80%. The holes contemplated for the tape include, but are not limited to, the slits contemplated Karami et al. in U.S. Patent Nos. 5,244,457 and 5,308,313, or by Gilman in U.S. Patent Nos. 5,056,510 and 5,106,362, the disclosures of which are incorporated herein by reference. However, the holes of the present invention need not be slits. Neither do the holes of the invention need to force "tortuous flow" of exudate. In a preferred embodiment, the holes of the invention shall not be slits or otherwise designed to force tortuous flow.

Preferably, the adhesive-coated face of the film will be uniformly coated with hydrocolloid adhesive. Accordingly, in this embodiment there will be no portions of the coated face of the film that lack adhesive.

The holes on the tape of this embodiment are uniformly spaced. Preferably, for any line in the plane of the film, no more than about 50% of the line will intersect a hole. More preferably, no more than about 30% of the line will intersect the line, still more preferably, no more than about 20%.

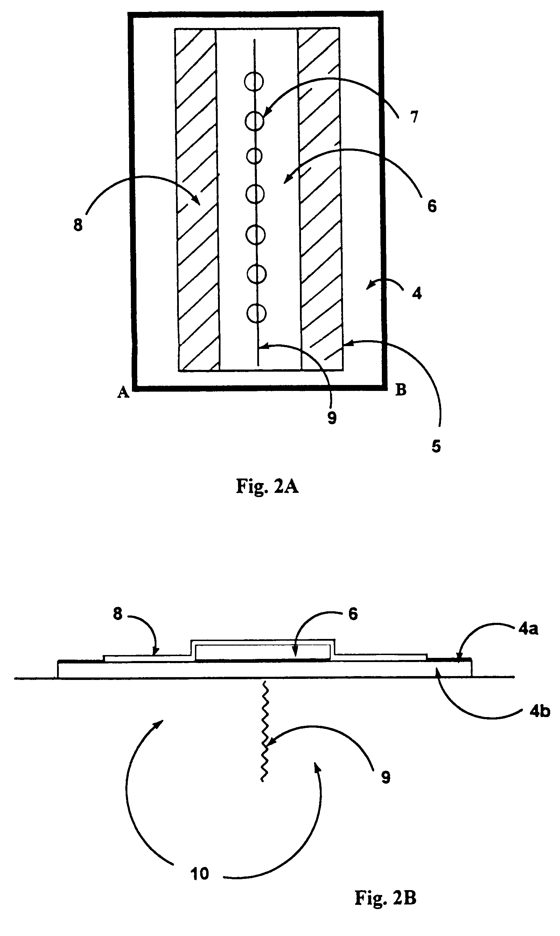

An absorbent dressing, containing an absorbent such as cotton, hydrophilic gels, gauze, alginate, etc., may be placed over the holes of the closure tape to absorb exudate. Preferably, the absorbent dressing will have an adhesive portion to adhere it in position over the exudate-releasing holes. The absorbent portion is generally substantially rectangular in shape, with adhesive portions extending from at least two opposing sides of the rectangular shape. Preferably, the face of the elastic film of the closure tape opposite from that covered with adhesive is surface-treated to allow facile application and removal of the adhesive portion of the absorbent dressings. Preferably, the non-adhesive face is surface treated with a siliconizing agent such as polysiloxane (which is available, for instance, from General Electric, Schenectady, NY). By this device, new absorbent dressings can be facilely adhered to or removed from the closure tape situated over a skin breach, without disturbing the closure tape, the wound or the skin adjacent to the closure tape. See Figure 2.

In Figure 2A, top view of the combination dressing, having both a closure tape (4) and an absorbent dressing (5) is illustrated. The absorbent (6) is situated over the holes (7) of the closure tape. The adhesive portion of the absorbent dressing (8) extends from two sides of the absorbent and is situated over the elastic film of the closure tape.

In Figure 2B, a side perspective along axis A-B of the dressing of Figure 2A is shown. The closure tape (4) includes a film layer (4a) and an hydrocolloid adhesive layer (4b). The skin breach (9) is situated under the absorbent (6) and separates two portions of skin tissue (10).

When a breach of the skin is deep enough, e.g., when it extends into fat or muscle layers, it may be necessary to use subcutaneous sutures in conjunction with the closure tape of the invention to sufficiently stabilize the wound, as will be recognized by a physician of ordinary skill. The use of subcutaneous sutures in conjunction with the invention is within the scope of the invention.

Those of ordinary skill will recognize the types of incisions or other skin breaches that have ordinarily been treated with sutures. Such breaches include breaches of deep partial thickness and of about full thickness. Deeper breaches are, of course, also included.

Use of the tape to close a skin breach can also include leaving the applied tape in place for a period of time sufficient to control exudate production. Preferably, the tape will be left in place for a period of time sufficient to permit epithelialization. Still more preferably, the tape will be left in place until the underlying epithelium has matured sufficiently so that the cell layer to which the film is adhered begins to slough off. Alternately, the tape may be left in place for at least about two days, preferably about 4 days, more preferably about 7 days, yet more preferably about 14 days.

The invention is illustrated by way of the following non-limiting examples.

### Example 1

Use of four different means for closing a breach of the skin were compared using a swine model. The breaches were full thickness incisions extending to, but not breaching the subcutaneous fat layer. The first means was suturing with a 3-0 silk, with the sutured wound left exposed to environment. The second means was suturing, with the sutured wound occluded with a hydrocolloid ("HCD") occlusive dressing of the type marketed as "DuoDerm Extra Thin®" (ConvaTec Division of Bristol-Myers Squibb, Inc., Princeton, New Jersey). The third means was closing the incision with a cyanoacrylate adhesive of the type marketed as "Nexabrand S/C®" (TriPoint Med, Raleigh, NC) which is a tissue glue. These cyanoacrylate adhesives are packaged as pre-polymers. When applied to a wound, the pre-polymers polymerize due to exposure to air and moisture. The polymerizate is hydrophobic, and thus lacks the capacity to absorb moisture. The fourth means was closing the incision with a HCD occlusive dressing, of the kind marketed as DuoDerm Extra Thin®, a use within the present invention.

Three, five way cross Yorkshire swine of approximately 15 kg were pre-anesthetized with an intra-muscular injection of Ketamine HCl (100 mg/ml). A surgical plane of anesthesia was inducted with Halothane™ 5% delivered by inhalation, NO₂ at 1.5 L/min and oxygen of 2.5 L/min. Anesthesia was maintained with 3.0% Halothane, NO₂ at 2.5 L/min and O₂ at 2.5 L/min.

Four full-thickness incisions of 5.0 cm length were made to each flank (for a total of 24 incisions), using a #10 scalpel. The incisions were placed 4 cm from and parallel to the vertebral column. Each incision was 0.4 cm deep and separated from adjacent incisions by 3 cm of intact skin. These incisions were full-thickness, extending through the panniculus carnosis, but did not breach the subcutaneous fat layer. Prior to closing the incision, hemostasis was achieved with gauze tamponade.

Suturing was accomplished by conventional means. The tissue glue and HCD occlusive dressing were carefully applied to align the edges of the incision. One of each treatment was applied to one of the four incisions on each side of a swine. Extreme care was needed with the Nexaband product (tissue glue) to avoid deposition of cyanoacrylate polymer within the incision.

For two of the swine, the adhesive dressings, when used, were left in place for seven days. The third swine had its dressing replaced on day two.

After seven days, the gross wound pathologies were as follows:

| WOUND CLOSURE | INFLAMMATION | SCAR/EDEMA PRESENT |
|---|---|---|
| Sutured Air Exposed | 1/6 | 6/6 |
| Sutured Occluded | 3/6 | 6/6 |
| Tissue Glue | 1/6 | 3/6* |
| HCD Dressing | 1/6 | 0/6 |

| | | |
|---|---|---|
| *The excess scarring for two incisions may have been due to the deposition of cyanoacrylate into the incisions. These two incisions were partially agape on day seven. | | |

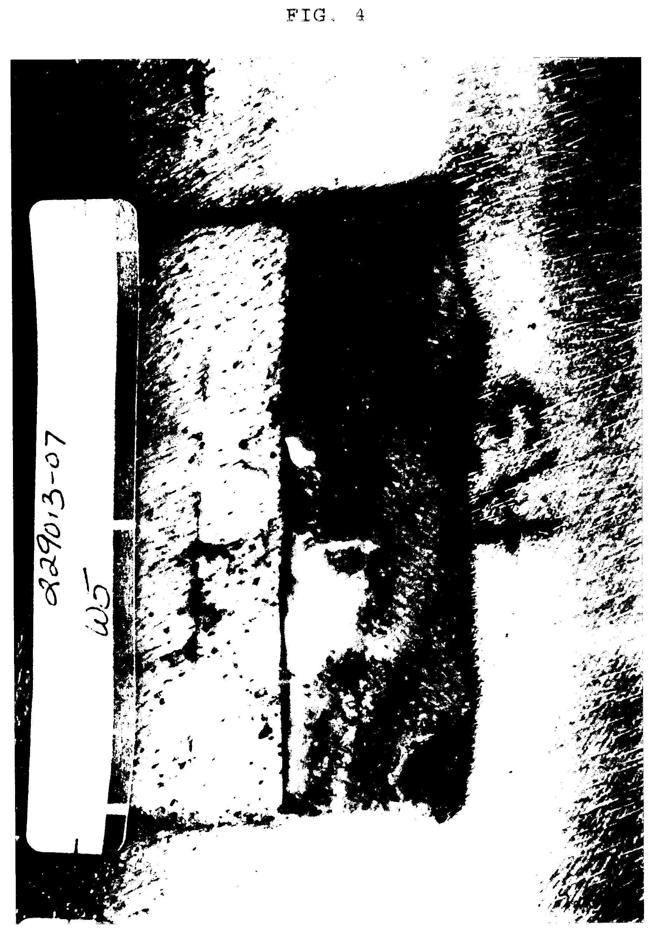

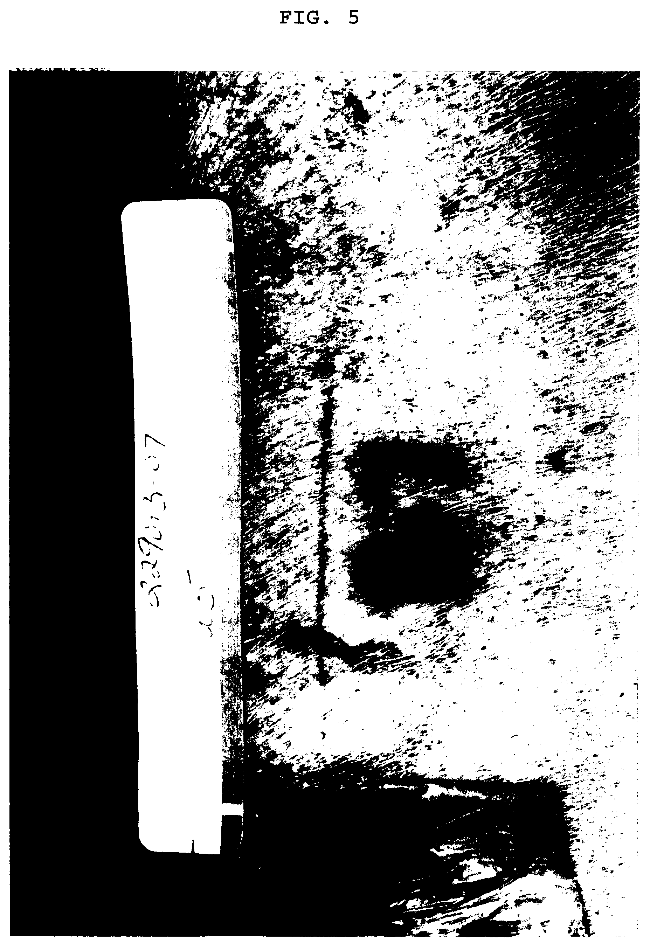

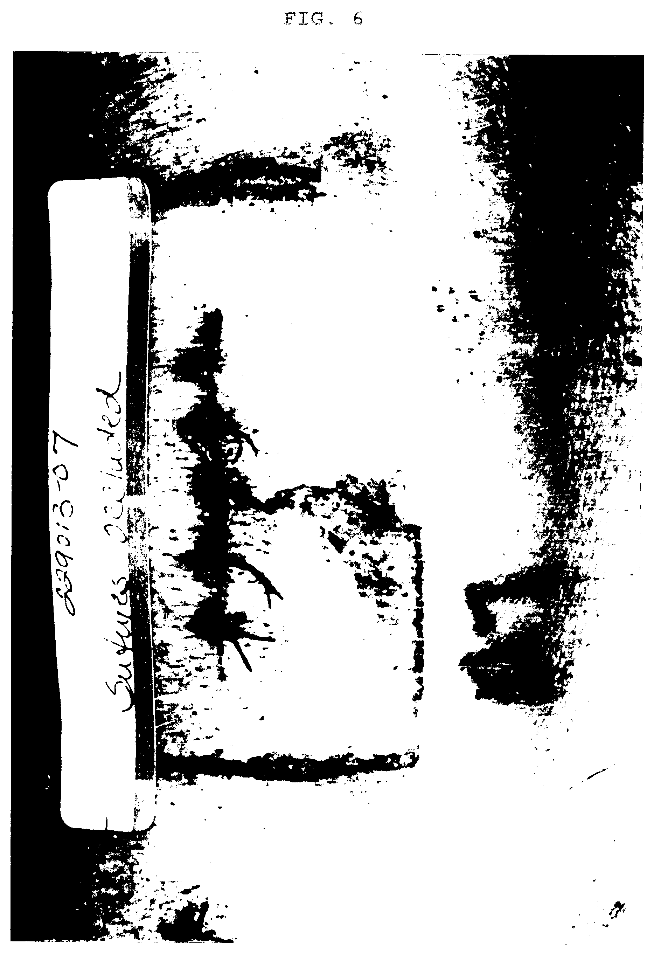

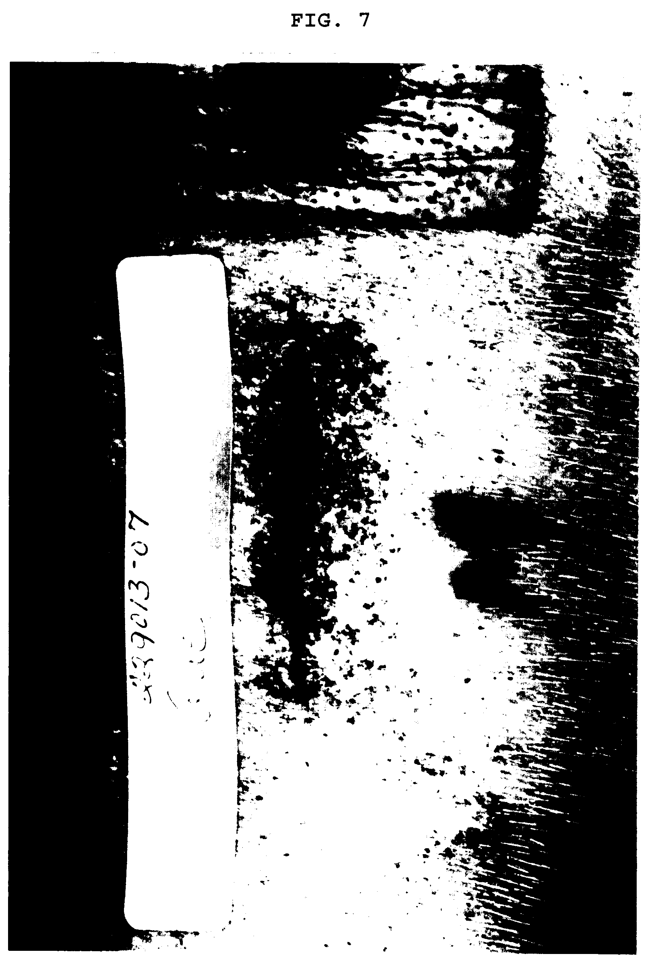

Representative photos of the closed incisions at day seven are displayed in Figures 4-7. Figure 4 shows a photograph of an incision covered with a HCD occlusive dressing. Figure 5 shows an incision after an HCD occlusive dressing has been pulled away. Figure 6 shows a healed incision that had been closed with an HCD occlusive dressing. Figure 7 shows an incision that was treated with sutures and a HCD occlusive dressing. Figure 8 shows an incision that was treated with tissue glue. These photographs graphically document the improved healing, with negligible scarring, obtained through use of the invention.

### Example 2

This example is directed to preparing an adhesive made having the following composition:

| | Percent by weight |
|---|---|
| Polyisobutylene (Vistanex LM-MH)* | 10.2 |
| Styrene-isoprene-styrene copolymer (Kraton 1107) | 12.6 |
| Mineral oil | 25.1 |
| Zinc dibutyldithiocarbamate (Butyl Zimate) | 0.06 |
| Tackifier (Pentalyn H) | 8.94 |
| Sodium carboxymethylcellulose | 31.0 |
| Cross-linked sodium carboxymethylcellulose (AcDiSol) | 12.1 |

| | |
|---|---|
| * A listing of suppliers follows Example 3 below. | |

The mineral oil (187.5 g), polyisobutylene (75.8 g), Kraton 1107 (92.8 g) and Butyl Zimate (0.4 g) were combined in a sigma blade mixer with heating (to about 115°C) and agitation for approximately one to three hours. Then, while mixing continued, the mixture was allowed to cool to about 100°C and sodium carboxymethylcellulose (89.1 g) and Pentalyn H (66.1 g) were added. Mixing was continued until a homogeneous mass resulted (after about 30 more minutes). The mass was allowed to cool and then it was flattened to the desired thickness. Silicone coated release paper was applied to both sides. After one of the release papers is removed, the adhesive layer can be applied to another surface, such as a polymeric, elastic film.

### Example 3

Compositions 2 - 25 were prepared following the procedure of Example 2, except that the components of the compositions, on a weight percent basis, were as listed in the following tables:

| Ingredient | 2 | 3 | 4 | 5 | 6 | 7 | 8 | 9 | 10 | 11 | 12 | 13 |
|---|---|---|---|---|---|---|---|---|---|---|---|---|
| Polyisobutylene (Vistanex LM-MH) | 11.68 | 11.68 | 7.75 | 5.9 | 6.7 | --- | 10 | 15 | 13 | --- | 15 | 15 |
| Polyisobutylene (Vistanex LM-MS) | --- | --- | --- | --- | --- | 10.8 | --- | --- | --- | 15 | --- | --- |
| Guar Gum | --- | --- | --- | --- | --- | 26.0 | --- | --- | --- | --- | --- | |
| Locust Bean Gum | --- | --- | -- | --- | --- | --- | --- | 20 | --- | --- | --- | --- |
| Pectin | --- | --- | --- | --- | --- | --- | 10 | --- | 10 | --- | --- | --- |
| Karaya | --- | --- | --- | --- | --- | --- | --- | --- | --- | 15 | --- | |
| Gelatin | --- | --- | --- | --- | --- | --- | 10 | --- | --- | --- | --- | --- |
| Sodium carboxymethylcellulose | 31.74 | 28.24 | 25.1 | 26.3 | 21.9 | --- | 11 | --- | 17 | 15 | 20 | 20 |
| Cross-linked sodium Carboxymethylcellulose | 12.34 | 12.34 | 9.1 | 10.5 | 8.0 | --- | --- | 12 | 12 | 8 | 12 | --- |
| Starch-acrylonitrile Graft copolymer (Grain Processing Corp. Polymer 35-A-100) | --- | --- | --- | --- | --- | 12.0 | --- | --- | --- | --- | --- | 10 |
| Coss-linked dextran (Sephadex CM-C50) | --- | --- | --- | --- | --- | --- | 10 | --- | --- | --- | --- | --- |
| Mineral Oil | 23.4 | 26.86 | 30.4 | 31.5 | 40.5 | 28.5 | 20 | 30 | 25 | 24 | 27 | 35 |
| S-1-S copolymer (Kraton 1107) | 11.68 | 11.68 | 19.2 | 16.7 | 13.2 | 15.0 | 20 | --- | 15 | --- | 18 | 12 |
| S-B-S coplymer (Kraton 1102) | --- | --- | --- | --- | --- | --- | --- | 15 | --- | 13 | --- | --- |
| Tackifier (Pentalyn H) | 9.10 | 9.14 | 12.3 | 8.9 | 9.6 | 7.5 | 8.99 | 7.98 | 7.98 | 9.99 | 7.98 | 7.98 |
| Antioxidant | 0.06 | 0.06 | 0.15 | 0.2 | 0.1 | 0.2 | 0.01 | 0.02 | 0.02 | 0.01 | 0.02 | 0.02 |
| Butyl Rubber (Grade 065) | --- | --- | --- | --- | --- | --- | --- | --- | --- | --- | --- | --- |

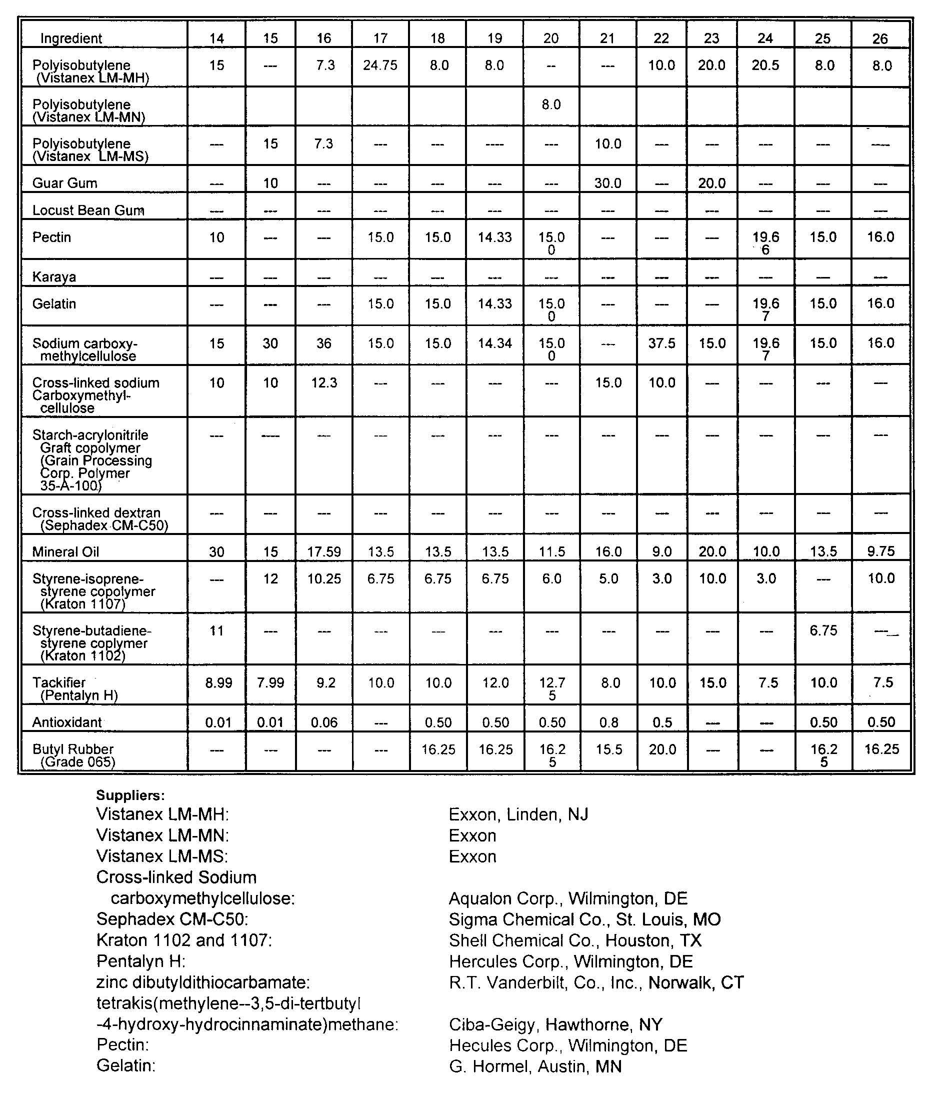

## Claims

1. Use of a tape comprising :
(i) a polymeric elastic film that is substantially impermeant to microorganisms, and
(ii) a hydrocolloid adhesive coating on one face of the film and having fluid absorbing capacity,
for the manufacture of a closure tape for use in closing a breach of the skin of the type that has typically been closed with sutures.

2. Use of a tape as claimed in claim 1 wherein said hydrocolloid adhesive is capable of swelling to incorporate at least 0.03 g of water per mil of thickness and cm² of area while retaining its adhesive tack.

3. Use of a tape as claimed in claim 2 wherein said hydrocolloid is capable of swelling to incorporate at least 0.05 g of water per mil of thickness and cm² of area while retaining its adhesive tack.

4. Use of a tape as claimed in claim 1, wherein the hydrocolloid coating comprises a substantially homogeneous mixture on a percent weight basis of from about 5% to about 30% by weight of one or more polyisobutylenes or a blend of one or more polyisobutylenes and butyl rubber, from about 3.0% to about 20% by weight of one or more styrene radial or block type copolymers, from about 8.0% to about 40% by weight of mineral oil, from about 15% to about 65% by weight of one or more water soluble hydrocolloid gums, up to about 15% by weight of one or more water swellable cohesive strengthening agents provided that said water soluble hydrocolloid gums and said water swellable cohesive strengthening agents together are present at from about 15% to about 65% by weight of said composition, and from about 7.5% to about 15% by weight of a tackifier.

5. Use of a tape as claimed in claim 4, wherein the water soluble hydrocolloid is selected from the group consisting of sodium carboxymethylcellulose, pectin, gelatin, guar gum, locust bean gum, gum karaya, and mixtures thereof and said water swellable cohesive strengthening agent is selected from the group consisting of cross-linked sodium carboxymethylcellulose, starch-acrylonitrile graft copolymers, and cross-linked dextran.

6. Use of a tape as claimed in claim 4 or claim 5, wherein the styrene copolymer is a styrene-isoprene-styrene or a styrene-butadiene-styrene block polymer.

7. Use of a tape as claimed in claim 6, wherein the styrene copolymer is a styrene-isoprene-styrene block copolymer.

8. Use of a tape as claimed in any of claims 4 to 7, wherein said polyisobutylenes are one or more low molecular weight polyisobutylenes.

9. Use of a tape as claimed in any preceding claim, wherein the hydrocolloid coating comprises a substantially homogeneous mixture on a percent weight basis of from about 5% to about 15% by weight of one or more low molecular weight polyisobutylenes or a blend of one or more low molecular weight polyisobutylenes and butyl rubber, from about 10% to about 20% by weight of a styrene block copolymer selected from the group consisting of styrene-isoprene-styrene and styrene-butadiene-styrene block copolymers, from about 20% to about 40% by weight of mineral oil, from about 30% to about 60% by weight of a water soluble hydrocolloid selected from the group consisting of sodium carboxymethylcellulose, pectin, gelatin, guar gum, locust bean gum, gum karaya, and mixtures thereof, up to about 15% by weight of a water swellable cohesive strengthening agent selected from the group consisting of cross-linked sodium carboxymethylcellulose, starch-acrylonitrile graft copolymer, and cross-linked dextran provided that said water soluble hydrocolloids and said water swellable cohesive strengthening agents are together present at from about 30% to about 65% by weight of said composition, from about 7.5% to about 10% by weight of a tackifier, and up to about 0.5% of an antioxidant.

10. Use of a tape as claimed in claim 9, wherein the styrene copolymer is a styrene-isoprene-styrene block copolymer.

11. Use of a tape as claimed in claim 10, wherein the tackifier is a pentaerythritol ester of rosin.

12. Use of a tape as claimed in claim 11, wherein the antioxidant is tetrakis(methylene--3,5-di-tertbutyl-4-hydroxy-hydrocinnaminate)methane.

13. A closure tape useful for closing wounds that produce substantial amounts of exudate comprising
(a) a polymeric elastic film that is substantially impermeant to microorganisms, wherein said film has a plurality of uniformly distributed holes, and
(b) a hydrocolloid adhesive coating on a face of said film comprising a substantially homogeneous mixture on a percent weight basis of from about 5% to about 30% by weight of one or more polyisobutylenes or a blend of one or more polyisobutylenes and butyl rubber, from about 3.0% to about 20% by weight of one or more styrene radial or block type copolymers, from about 8.0% to about 40% by weight of mineral oil, from about 15% to about 65% by weight of one or more water soluble hydrocolloid gums, up to about 15% by weight of one or more water swellable cohesive strengthening agents provided that said water soluble hydrocolloid gums and said water swellable cohesive strengthening agents together are present at from about 15% to about 65% by weight of said composition, and from about 7.5% to about 15% by weight of a tackifier.
